# EUROPEAN PATENT APPLICATION

(11) **EP 4 783 174 A1**
(43) Date of publication of application: **29.07.2026**
(21) Application number: 24932680.2
(22) Date of filing: 30.12.2024
(51) Int. Cl.: G16B 50/40

(54) **GENE SEQUENCING DATA ANALYSIS METHOD AND APPARATUS, COMPUTING DEVICE AND READABLE STORAGE MEDIUM**

(30) Priority: 29.03.2024 CN 202410385100
(71) Applicant: Sansure Biotech Inc., Changsha, Hunan 410205 (CN)
(72) Inventor: YANG, Li, Changsha, Hunan 410205 (CN); DENG, Xiaolong, Changsha, Hunan 410205 (CN); GUO, Juncheng, Changsha, Hunan 410205 (CN); DAI, Lizhong, Changsha, Hunan 410205 (CN)
(74) Representative: Murgitroyd & Company
(86) International application number: PCT/CN2024/143848
(87) International publication number: WO 2025/200669

(57) **Abstract**

The embodiments of the present application belong to the field of data processing. Provided are a gene sequencing data analysis method and apparatus, a computing device and a readable storage medium. The gene sequencing data analysis method is applied to a user equipment. The gene sequencing data analysis method comprises: encoding gene sequencing data of a user to obtain a plurality of gene sequencing plaintexts corresponding to the gene sequencing data (S110); using a public key to encrypt each of the gene sequencing plaintexts to obtain a plurality of gene sequencing ciphertexts (S120); sending the gene sequencing ciphertexts to a server (S130); receiving a gene sequencing data analysis result ciphertext sent by the server (S140); and using a private key to decrypt the gene sequencing data analysis result ciphertext to determine a gene sequencing data analysis result of the user (S150). The user equipment sends the plurality of encrypted gene sequencing ciphertexts to the server, thereby avoiding leakage of user privacy. The server uses a preset sparse model to perform data analysis and process the gene sequencing ciphertexts to be tested, thereby improving the gene sequencing data analysis efficiency.

## Description

### CROSS-REFERENCE TO RELATED APPLICATIONS

The present application claims priority to Chinese patent application No. 202410385100.4 filed on March 29, 2024, which is hereby incorporated by reference in its entirety.

### TECHNICAL FIELD

The present disclosure generally relates to the field of data processing technologies, and in particular to a gene sequencing data analysis method, an apparatus, a computing device, and a readable storage medium.

### BACKGROUND

With rapid advancement of medical diagnostic technology, remote diagnosis has been widely adopted. Users may utilize user equipment to send personal information, including gene sequencing data, to a remote diagnosis device. The remote diagnosis device analyzes the received gene sequencing data and returns a gene sequencing data analysis result to the user equipment. The user receives the gene sequencing data analysis result through the user equipment to determine whether individual is suffering from a specific disease, thereby preventing the users with diseases from missing an optimal treatment opportunity.

When a local medical institution is unable to accurately analyze a case of the user, the remote diagnosis equipment may be utilized to perform remote analysis for the gene sequencing data of the user, thereby determining whether the user is suffering from a disease and improving efficiency of analyzing and processing the gene sequencing data. However, the remote analysis of the gene sequencing data requires users to provide a large amount of medical information, such as the gene sequencing data to be analyzed and processed, and to send the medical information to the remote diagnosis device. The medical information of the user may easily be leaked. Furthermore, when the remote diagnosis device returns the gene sequencing data analysis result, the analysis result may also easily be leaked, leading to a risk of leakage of user privacy when performing the remote analysis of the genetic sequencing data.

### SUMMARY

The purpose of embodiments of the present disclosure is to provide a gene sequencing data analysis method, an apparatus, a computing device, and a readable storage medium. The gene sequencing data analysis method is used to solve a problem of easy leakage of user privacy when performing a remote analysis of gene sequencing data.

To achieve the above-mentioned purpose, in a first aspect, the present disclosure provides a gene sequencing data analysis method applied to a user equipment. The gene sequencing data analysis method includes:
encoding gene sequencing data of a user to obtain a plurality of gene sequencing plaintexts corresponding to the gene sequencing data;
encrypting each of the plurality of gene sequencing plaintexts using a public key to obtain a plurality of gene sequencing ciphertexts;
sending the plurality of gene sequencing ciphertexts to a server, where the server is configured to input the plurality of gene sequencing ciphertexts into a preset sparse model to obtain a gene sequencing data analysis result ciphertext;
receiving a gene sequencing data analysis result ciphertext sent by the server; and
decrypting the gene sequencing data analysis result ciphertext using a private key to determine a gene sequencing data analysis result of the user.

In the embodiments of the present disclosure, the gene sequencing data analysis method further includes:
generating, based on a ciphertext polynomial degree and a bit length of a ciphertext modulus of a homomorphic encryption, a security parameter; and
generating the public key and the private key according to the security parameter.

In the embodiments of the present disclosure, the encoding the gene sequencing data of the user to obtain the plurality of gene sequencing plaintexts corresponding to the gene sequencing data, includes:
determining an encoding dimension corresponding to each of the plurality of gene sequencing ciphertexts according to the ciphertext polynomial degree; and
encoding, based on the encoding dimension and a dimension of the gene sequencing data of the user, the gene sequencing data, to obtain the plurality of gene sequencing plaintexts.

In the embodiments of the present disclosure, the gene sequencing data analysis method further includes:
when a dimension of the plurality of gene sequencing plaintext is less than the encoding dimension, performing data padding on the plurality of gene sequencing plaintext until the dimension of the plurality of gene sequencing plaintext equals the encoding dimension.

In the embodiments of the present disclosure, a dimension of model parameters of the preset sparse model equals a dimension of the gene sequencing data, and the model parameters include model sub-parameters corresponding to each of the plurality of gene sequencing plaintexts; and
the preset sparse model is configured to obtain a plurality of result ciphertexts respectively according to the model sub-parameters and the plurality of gene sequencing ciphertexts, and sum the plurality of result ciphertexts to obtain the gene sequencing data analysis result ciphertext.

In the embodiments of the present disclosure, the decrypting the gene sequencing data analysis result ciphertext using the private key to determine the gene sequencing data analysis result of the user, includes:
decrypting the gene sequencing data analysis result ciphertext using the private key to obtain a plaintext vector corresponding to the gene sequencing data analysis result, where the plaintext vector comprises a plurality of components;
obtaining any one of the plurality of components from the plaintext vector;
when a numerical value of the component is positive, determining that the gene sequencing data analysis result of the user indicates the genetic sequencing data is abnormal; and
when the numerical value of the component is negative, determining that the gene sequencing data analysis result of the user indicates the genetic sequencing data is not abnormal.

In the embodiments of the present disclosure, the gene sequencing data analysis method further includes:
generating a data analysis and processing document of the user according to the gene sequencing data analysis result of the user; and
converting the data analysis and processing document into a QR code image and generating a display interface for the QR code image.

In a second aspect, the present disclosure provides a gene sequencing data analysis method applied to a server. The gene sequencing data analysis method includes:
receiving a plurality of gene sequencing ciphertexts sent by a user equipment;
inputting the plurality of gene sequencing ciphertexts into a preset sparse model to obtain a gene sequencing data analysis result ciphertext; wherein the preset sparse model is configured to process a target gene sequencing ciphertext and output the gene sequencing data analysis result ciphertext, and the target gene sequencing ciphertext is a gene sequencing ciphertext including gene locus data to be detected in the plurality of gene sequencing ciphertexts; and
sending the gene sequencing data analysis result ciphertext to the user equipment, so that the user equipment decrypts the gene sequencing data analysis result ciphertext to determine a gene sequencing data analysis result of a user.

In a third aspect, the present disclosure provides a gene sequencing data analysis apparatus applied to a user equipment, where the gene sequencing data analysis apparatus includes:
a data encoding module, configured to encode gene sequencing data of a user to obtain a plurality of gene sequencing plaintexts corresponding to the gene sequencing data;
a plaintext encryption module, configured to encrypt each of the plurality of gene sequencing plaintexts using a public key to obtain a plurality of gene sequencing ciphertexts;
a ciphertext sending module, configured to send the plurality of gene sequencing ciphertexts to a server, wherein the server is configured to input the plurality of gene sequencing ciphertexts into a preset sparse model to obtain a gene sequencing data analysis result ciphertext;
a ciphertext receiving module, configured to receive the gene sequencing data analysis result ciphertext sent by the server; and
a result determination module, configured to decrypt the gene sequencing data analysis result ciphertext using a private key to determine a gene sequencing data analysis result of the user.

In a fourth aspect, the present disclosure provides a gene sequencing data analysis apparatus applied to a server, where the gene sequencing data analysis apparatus includes:
a gene sequencing ciphertext receiving module, configured to receive a plurality of gene sequencing ciphertexts sent by a user equipment;
a gene sequencing ciphertext input module, configured to input the plurality of gene sequencing ciphertexts into a preset sparse model to obtain a gene sequencing data analysis result ciphertext; wherein the preset sparse model is configured to process a target gene sequencing ciphertext and output the gene sequencing data analysis result ciphertext, and the target gene sequencing ciphertext is a gene sequencing ciphertext including gene locus data to be detected in the plurality of gene sequencing ciphertexts; and
a result ciphertext sending module, configured to send the gene sequencing data analysis result ciphertext to the user equipment, so that the user equipment decrypts the gene sequencing data analysis result ciphertext to determine the gene sequencing data analysis result of the user.

In a fifth aspect, the present disclosure further provides a computing device, including:
a memory, configured to store instructions; and
a processor, configured to invoke the instructions from the memory and implement the above-mentioned gene sequencing data analysis method when executing the instructions.

In a sixth aspect, the present disclosure further provides a machine-readable storage medium. The machine-readable storage medium stores instructions and the instructions are used to cause a machine to execute the above-mentioned gene sequencing data analysis method.

The present disclosure provides a gene sequencing data analysis method applied to a user equipment. The gene sequencing data analysis method includes: encoding gene sequencing data of a user to obtain a plurality of gene sequencing plaintexts corresponding to the gene sequencing data; encrypting each of the plurality of gene sequencing plaintexts using a public key to obtain a plurality of gene sequencing ciphertexts; sending the plurality of gene sequencing ciphertexts to a server; receiving a gene sequencing data analysis result ciphertext sent by the server; and decrypting the gene sequencing data analysis result ciphertext using a private key to determine a gene sequencing data analysis result of the user. When performing gene sequencing data analysis for the user, the user equipment sends the plurality of encrypted gene sequencing ciphertexts, thereby preventing a leakage of the gene sequencing data of the user. The server utilizes a preset sparse model for data analysis, does not need to process all the gene sequencing ciphertexts, but processes gene sequencing ciphertexts to be detected, thereby improving computational efficiency of the model, and efficiency of gene sequencing data analysis. Furthermore, even when all the gene sequencing ciphertexts sent by the user equipment are intercepted and decrypted by a third-party device, since the gene sequencing ciphertexts to be detected for the user may not be identified, user privacy leakage during remote gene sequencing data analysis is prevented.

Other features and advantages of the embodiments of the present disclosure may be described in detail in the following section of detailed description of the embodiments.

### BRIEF DESCRIPTION OF THE DRAWINGS

Accompanying drawings are provided to further enhance an understanding of embodiments of the present disclosure and form part of the specification, and are used together with the following detailed description of the embodiments to explain the embodiments of the present disclosure, and do not constitute a limitation of the embodiments of the present disclosure. In the accompanying drawings:
FIG. 1 is a first flowchart of a gene sequencing data analysis method according to an embodiment of the present disclosure.
FIG. 2 is an application example diagram of a user equipment according to an embodiment of the present disclosure.
FIG. 3 is a second flowchart of a gene sequencing data analysis method according to an embodiment of the present disclosure.
FIG. 4 is a first structural schematic diagram of a gene sequencing data analysis apparatus according to an embodiment of the present disclosure.
FIG. 5 is a second structural schematic diagram of a gene sequencing data analysis apparatus according to an embodiment of the present disclosure.

### DETAILED DESCRIPTION OF THE EMBODIMENTS

Specific embodiments of the present disclosure may be described in detail below in combination with accompanying drawings in the embodiments of the present disclosure. It is understandable that the specific implementations described herein are only for illustrating and explaining the embodiments of the present disclosure, and are not intended to limit the embodiments of the present disclosure.

Components described and illustrated in the accompanying drawings herein for the embodiments of the present disclosure may be arranged and designed in different configurations. Therefore, the detailed description of the embodiments of the present disclosure provided in the accompanying drawings is not intended to limit a scope of the claimed disclosure, but merely represents selected embodiments of the present disclosure. All other embodiments obtained by those skilled in the art without creative efforts may fall within a protection scope of the present disclosure.

In the following description, terms 'include' and 'have' and the cognates as used in various embodiments of the present disclosure are intended merely to denote specific features, numbers, steps, operations, elements, components, or combinations thereof, and may not be understood as first excluding the existence or possibility of adding the one or more other features, numbers, steps, operations, elements, components, or combinations thereof.

Furthermore, terms 'first', 'second', and 'third' and the like are used for distinguishing descriptions and may not be understood as indicating or implying relative importance.

Unless otherwise defined, all terms (including technical and scientific terms) used herein have the same meanings as commonly understood by those skilled in the art belonging to the various embodiments of the present disclosure. The terms (e.g., terms defined in commonly used dictionaries) may be interpreted as having meanings consistent with contextual meanings in a relevant technical field and may not be interpreted in an idealized or overly formal meanings unless expressly so defined in the various embodiments of the present disclosure.

### First Embodiment

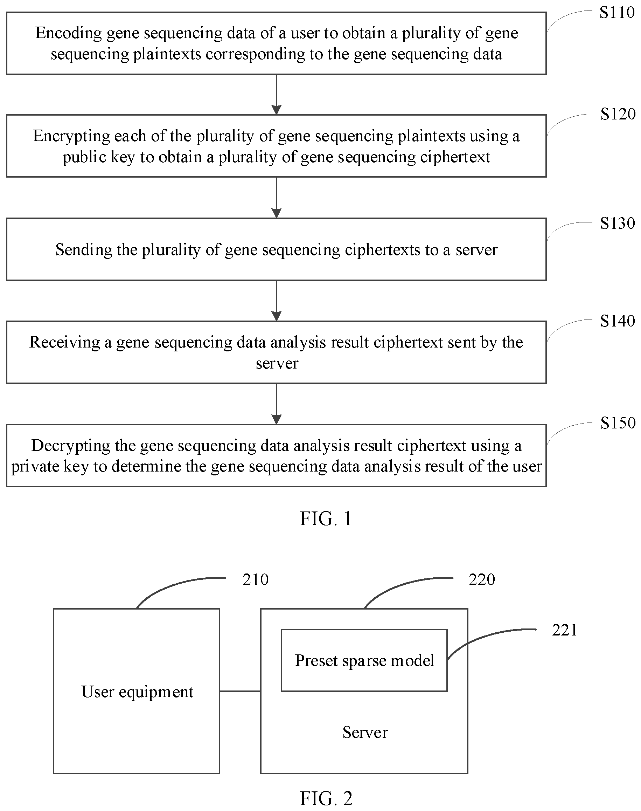

Referring to FIG. 1, FIG. 1 is a first flowchart of a gene sequencing data analysis method according to an embodiment of the present disclosure. The gene sequencing data analysis method in FIG. 1 is applied to a user equipment. The gene sequencing data analysis method includes
Step S110, encoding gene sequencing data of a user to obtain a plurality of gene sequencing plaintexts corresponding to the gene sequencing data.

Gene sequencing is a technique used to determine the Deoxyribonucleic Acid (DNA) sequence within a genome of an organism. The gene sequencing is performed on the user to obtain the gene sequencing data of the user. A gene locus refers to a position with a specific genetic variation on the genome of the user. The gene sequencing data includes the gene locus data of the user, thereby enabling analysis and processing of the gene sequencing data to obtain a gene sequencing data analysis result. The gene sequencing data of the user is encoded to obtain the plurality of gene sequencing plaintexts corresponding to the gene sequencing data, that is, the gene sequencing data is encoded into the plurality of gene sequencing plaintexts.

Step S120, encrypting each of the plurality of gene sequencing plaintexts using a public key to obtain a plurality of gene sequencing ciphertexts.

When performing gene sequencing data analysis for the user, the user equipment generates a public key and a private key. The public key is used to encrypt data, and the private key is used to decrypt data. Each gene sequencing plaintext is encrypted using the public key to obtain the gene sequencing ciphertext corresponding to each gene sequencing plaintext. By encoding the gene sequencing data into the plurality of gene sequencing plaintexts and encrypting each gene sequencing plaintext separately, a need to simultaneously process data with excessively large numerical ranges is avoided, thereby improving data processing efficiency. And encrypting the gene sequencing plaintexts into gene sequencing ciphertexts may protect privacy of the user and prevent leakage of the gene sequencing data of the user.

Step S130, sending the plurality of gene sequencing ciphertexts to a server, where the server is configured to input the plurality of gene sequencing ciphertexts into a preset sparse model to obtain a gene sequencing data analysis result ciphertext.

Referring to FIG. 2, FIG. 2 is an application example diagram of a user equipment according to an embodiment of the present disclosure.

It is understandable that a type of a user equipment 210 is set according to actual requirements, the type may be a mobile terminal, a Personal Computer (PC), etc., and is not limited herein. A server 220 includes a preset sparse model 221. The user equipment 210 communicates with the server 220, and the user equipment 210 sends a plurality of obtained gene sequencing ciphertexts together to the server 220. The server 220 receives the encrypted gene sequencing ciphertexts, thereby preventing leakage of privacy of a user.

Since a plurality of gene sequencing plaintexts are obtained by encoding gene sequencing data, gene locus data of the user may be determined from the plurality of obtained gene sequencing plaintexts. The server 220 inputs the received gene sequencing ciphertexts into the preset sparse model 221, utilizes the preset sparse model 221 to process the gene sequencing ciphertexts, and thereby obtaining a gene sequencing data analysis result ciphertext according to the gene locus data within the gene sequencing ciphertexts.

In practical scenarios for determining the gene sequencing data analysis result of the user, analysis is typically performed for specific disease categories. A small amount of gene locus data to be detected that is related to the specific disease categories is processed and analyzed, and there is no need to process and analyze all the gene locus data. During communication process between the user equipment 210 and the server 220, the complete encrypted gene sequencing data is sent to the server 220. The different disease categories being analyzed may result in different gene sequencing ciphertexts to be detected. Compared to sending the gene locus data to be detected, even when all the gene sequencing ciphertexts sent by the user equipment 210 are intercepted and decrypted by a third-party device, since the gene sequencing ciphertexts to be detected for the user may not be identified, the disease category under analysis for the user may not be determined, thereby preventing the leakage of the privacy of the user.

Step S140, receiving a gene sequencing data analysis result ciphertext sent by the server.

The preset sparse model, due to that most of the model parameters are zero, even when the user device sends the plurality of genomic sequencing ciphertexts, the preset sparse model does not need to process all gene sequencing ciphertexts, but processes gene sequencing ciphertexts to be detected, thereby improving computational efficiency of the model, and efficiency of gene sequencing data analysis. After obtaining the gene sequencing data analysis result ciphertext, the server sends the gene sequencing data analysis result ciphertext to the user equipment. The user equipment receives the gene sequencing data analysis result ciphertext sent by the server, to determine the gene sequencing data analysis result of the user.

Step S150, decrypting the gene sequencing data analysis result ciphertext using the private key to determine the gene sequencing data analysis result of the user.

After receiving the gene sequencing data analysis result ciphertext sent by the server, the user equipment decrypts the gene sequencing data analysis result ciphertext using the private key to determine the gene sequencing data analysis result of the user. Since data sent and received by the user equipment are encrypted ciphertext data, user privacy leakage during gene sequencing data analysis is prevented.

In the embodiments of the present disclosure, the gene sequencing data analysis method further includes:
generating, based on a ciphertext polynomial degree and a bit length of a ciphertext modulus of a homomorphic encryption, a security parameter; and
generating the public key and the private key according to the security parameter.

It is understandable that an encryption method adopted is selected according to actual requirements, any homomorphic encryption method may be employed, and is not limited herein. For ease of understanding, Cheon Kim Kim Song (CKKS) homomorphic encryption is employed to generate the public key and the private key for encrypting each gene sequencing plaintext in the embodiments of the present disclosure. The adoption of the CKKS homomorphic encryption enables the preset sparse model to perform computations while the data is encrypted, thereby obtaining the gene sequencing data analysis result ciphertext. A result obtained after decrypting the gene sequencing data analysis result ciphertext is the same as a result obtained by performing computations on the unencrypted gene sequencing data. The ciphertext polynomial degree affects a depth of homomorphic operations performed by the user equipment. A lower ciphertext polynomial degree results in higher computational efficiency, but it may further lead to more complex operations corresponding to the ciphertext polynomial degree. The bit length of the ciphertext modulus affects a numerical range and a numerical precision represented by the gene sequencing ciphertext. A higher bit length of the ciphertext modulus results in higher numerical precision of the gene sequencing ciphertext, but it may further lead to an increased numerical range of the gene sequencing ciphertext, thereby affecting the computational efficiency.

It is understandable that a numerical value of the ciphertext polynomial degree and a numerical value of the bit length of the ciphertext modulus are set according to the actual requirements. The numerical values of the ciphertext polynomial degree and the numerical value of the bit length of the ciphertext modulus may be determined through homomorphic encryption security standards, and are not limited herein. For ease of understanding, in the embodiments of the present disclosure, the ciphertext polynomial degree is 8192, and the bit length of the ciphertext modulus is 60+40+40+60=200. Based on the ciphertext polynomial degree and the bit length of the ciphertext modulus of the CKKS homomorphic encryption, the generated security parameter is 128. Given the determined security parameter, the public key and private key are generated according to the security parameter and procedures specified by the CKKS homomorphic encryption.

In the embodiments of the present disclosure, the encoding the gene sequencing data of the user to obtain the plurality of gene sequencing plaintexts corresponding to the gene sequencing data, includes:
determining an encoding dimension corresponding to each of the plurality of gene sequencing ciphertexts according to the ciphertext polynomial degree; and
encoding, based on the encoding dimension and a dimension of the gene sequencing data of the user, the gene sequencing data, to obtain the plurality of gene sequencing plaintexts.

The encoding dimension corresponding to each of the plurality of gene sequencing ciphertexts is determined according to the ciphertext polynomial degree. In the embodiments of the present disclosure, the ciphertext polynomial degree is 8192, and the encoding dimension corresponding to each gene sequencing ciphertext is 8192/2 = 4096.

The gene sequencing data is encoded, based on the encoding dimension and the dimension of the gene sequencing data of the user, to obtain the plurality of gene sequencing plaintexts. Taking the dimension of the gene sequencing data as 342,669 as an example, after encoding the gene sequencing data, the number of the gene sequencing plaintexts obtained is 342669/4096 ≈ 84 segments. Compared to directly encrypting the complete gene sequencing data, encoding the gene sequencing data into the plurality of gene sequencing plaintexts and encrypting the plurality of gene sequencing plaintexts improves data processing efficiency.

In the embodiments of the present disclosure, the gene sequencing data analysis method further includes:
when a dimension of the plurality of gene sequencing plaintexts is less than the encoding dimension, performing data padding on the plurality of gene sequencing plaintexts until the dimension of the plurality of gene sequencing plaintexts equals the encoding dimension.

When encoding the gene sequencing data, the dimension of the last segment of gene sequencing plaintext may be less than the encoding dimension. Data padding is performed on the gene sequencing plaintext until the dimension of the gene sequencing plaintext equals the encoding dimension, ensuring that the dimension of each gene sequencing plaintext is the same. The padding data is set according to actual requirements and is not limited herein. For ease of understanding, in the embodiments of the present disclosure, the padding data is 0.

In the embodiments of the present disclosure, a dimension of model parameters of the preset sparse model equals the dimension of the gene sequencing data, and the model parameters include model sub-parameters corresponding to each of the plurality of gene sequencing plaintexts; and
the preset sparse model is configured to obtain a plurality of result ciphertexts respectively according to the model sub-parameters and the plurality of gene sequencing ciphertexts, and sum the plurality of result ciphertexts to obtain the gene sequencing data analysis result ciphertext.

The dimension of the model parameters of the preset sparse model equals the dimension of the gene sequencing data. That is, when the dimension of the gene sequencing data is 342,669, the dimension of the model parameters of the preset sparse model is also 342,669. In the embodiments of the present disclosure, 84 gene sequencing plaintexts are obtained. Therefore, the model parameters include model sub-parameters corresponding to each gene sequencing plaintext, that is, the number of model sub-parameters is 84.

The preset sparse model is configured to obtain the plurality of result ciphertexts respectively according to the model sub-parameters and the plurality of gene sequencing ciphertexts. The plurality of result ciphertexts is summed to obtain a ciphertext, that is, the gene sequencing data analysis result ciphertext. For ease of understanding, in the embodiments of the present disclosure, the preset sparse model is exemplified as a linear model: f(x) = w x + b, where f(x) is an output of the preset sparse model, x is the gene sequencing ciphertext, w and b are the model parameters of the preset sparse model, and a superscript T denotes a transpose of w. The preset sparse model, based on w and x, computes a plaintext-ciphertext vector inner product to obtain the result ciphertext zi corresponding to each gene sequencing plaintext, where i ranges from 1 to M/t, M is the dimension of the gene sequencing data, t is the encoding dimension corresponding to each gene sequencing ciphertext, x and w are both M-dimensional vectors, and b is a scalar. w is partitioned into M/t segments to obtain the model sub-parameter corresponding to each gene sequencing plaintext.

All zi are summed to obtain z, and z is then updated to obtain the gene sequencing data analysis result ciphertext as z+b. Since the plurality of model sub-parameters of the preset sparse model are zero, a large number of values of zi are zero. Only a small number of non-zero zi participate in a summation calculation. Computational load of summing the plurality of result ciphertexts is reduced, and the computational efficiency is improved, thereby enhancing the efficiency of gene sequencing data analysis.

In the embodiments of the present disclosure, the decrypting the gene sequencing data analysis result ciphertext using the private key to determine the gene sequencing data analysis result of the user, includes:
decrypting the gene sequencing data analysis result ciphertext using the private key to obtain a plaintext vector corresponding to the gene sequencing data analysis result, where the plaintext vector comprises a plurality of components;
obtaining any one of the plurality of components from the plaintext vector;
when a numerical value of the component is positive, determining that the gene sequencing data analysis result of the user indicates the genetic sequencing data is abnormal; and
when the numerical value of the component is negative, determining that the gene sequencing data analysis result of the user indicates the genetic sequencing data is not abnormal.

The gene sequencing data analysis result ciphertext is decrypted using the private key to obtain a plaintext vector corresponding to the gene sequencing data analysis result. The dimension of the gene sequencing data analysis result ciphertext equals the encoding dimension corresponding to the gene sequencing ciphertext, resulting in the plaintext vector including the plurality of components. The component is extracted from the plaintext vector, and any one component obtained from the plaintext vector serves as the gene sequencing data analysis result.

When a numerical value of the component is positive, it is determined that the gene sequencing data analysis result of the user indicates the genetic sequencing data is abnormal, thereby determining that the user is suffering from a disease. When the numerical value of the component is negative, it is determined that the gene sequencing data analysis result of the user indicates the genetic sequencing data is not abnormal, thereby determining that the user is not suffering from the disease. Since the gene sequencing data analysis result of the user is represented by the numerical value of the component, when the gene sequencing data analysis result ciphertext is intercepted and decrypted by a third-party device, the gene sequencing data analysis result does not include the specific disease category performed by an analysis, thereby preventing the leakage of the privacy of the user. Specifically, taking the gene sequencing data analysis result indicates the genetic sequencing data is abnormal as an example, it may only be determined that the user is suffering from the disease, but the specific disease category of the disease the user is suffering from may not be identified, thereby preventing the leakage of the privacy of the user.

In the embodiments of the present disclosure, the gene sequencing data analysis method further includes:
generating a data analysis and processing document of the user according to the gene sequencing data analysis result of the user; and
converting the data analysis and processing document into a QR code image and generating a display interface for the QR code image.

The data analysis and processing document of the user is generated according to the gene sequencing data analysis result of the user, thereby providing the gene sequencing data analysis result for the user through the data analysis and processing document. Specifically, when the gene sequencing data analysis result indicates the genetic sequencing data is abnormal, it is determined that the user is suffering from the disease. The data analysis and processing document may include the gene sequencing data analysis result and a treatment suggestion for the disease. Specifically, when the gene sequencing data analysis result of the user indicates the genetic sequencing data is not abnormal, it is determined that the user is not suffering from the disease. The data analysis and processing document may include the gene sequencing data analysis result and a prevention suggestion for the disease. It is understandable that the data analysis and processing document may further include other document information, such as prompts indicating a need for further diagnosis of the disease. The other document information is set according to actual requirements and is not limited herein.

The data analysis and processing document is converted into the QR code image and the display interface for the QR code image is generated. The users can read the QR code image in the display interface using personal devices such as mobile terminals. After user identity verification, the data analysis and processing document is displayed on the personal device, allowing the users to determine whether the user is suffering from the disease based on the gene sequencing data analysis result. It is understandable that the user equipment and the personal device of the user may be the same device or different devices. When the user equipment and the personal device are the same device, the display interface for the data analysis and processing document may be generated directly.

The present disclosure provides the gene sequencing data analysis method applied to the user equipment. The gene sequencing data analysis method includes: encoding the gene sequencing data of the user to obtain the plurality of gene sequencing plaintexts corresponding to the gene sequencing data; encrypting each of the plurality of gene sequencing plaintexts using the public key to obtain the plurality of gene sequencing ciphertexts; sending the plurality of gene sequencing ciphertexts to the server; receiving the gene sequencing data analysis result ciphertext sent by the server; and decrypting the gene sequencing data analysis result ciphertext using the private key to determine the gene sequencing data analysis result of the user. When performing gene sequencing data analysis for the user, the user equipment sends the plurality of encrypted gene sequencing ciphertexts, thereby preventing the leakage of the gene sequencing data of the user. And the server utilizes the preset sparse model for gene sequencing data analysis, does not need to process all the gene sequencing ciphertexts, but processes gene sequencing ciphertexts to be detected. Computational efficiency of the model is improved, thereby improving efficiency of gene sequencing data analysis. Furthermore, even when the gene sequencing ciphertexts sent by the user equipment are intercepted and decrypted by the third-party device, since the gene sequencing ciphertexts to be detected for the user may not be identified, user privacy leakage during remote gene sequencing data analysis is prevented.

### Second Embodiment

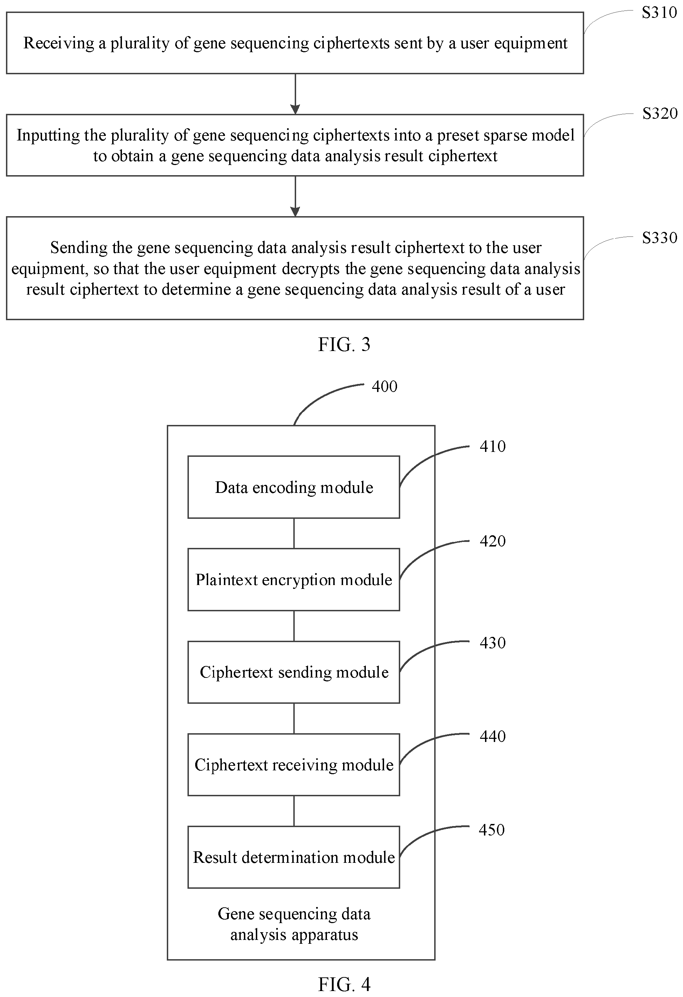

Referring to FIG. 3, FIG. 3 is a second flowchart of a gene sequencing data analysis method according to an embodiment of the present disclosure. The gene sequencing data analysis method in FIG. 3 is applied to a server. The gene sequencing data analysis method includes:
Step S310, receiving a plurality of gene sequencing ciphertexts sent by a user equipment.

The server communicates with the user equipment. When a user needs to perform remote gene sequencing data analysis, the user equipment encodes gene sequencing data of the user to obtain a plurality of gene sequencing plaintexts corresponding to the gene sequencing data. And the user equipment encrypts each of the plurality of gene sequencing plaintexts using a public key to obtain the plurality of gene sequencing ciphertexts; The user equipment sends the plurality of gene sequencing ciphertexts to the server. The server receives the plurality of gene sequencing ciphertexts sent by the user equipment, to obtain the gene sequencing data analysis result ciphertext through the server.

Step S320, inputting the plurality of gene sequencing ciphertexts into a preset sparse model to obtain the gene sequencing data analysis result ciphertext, wherein the preset sparse model is configured to process a target gene sequencing ciphertext and output the gene sequencing data analysis result ciphertext, and the target gene sequencing ciphertext is a gene sequencing ciphertext including gene locus data to be detected in the plurality of gene sequencing ciphertexts; and
the preset sparse model may be any preset model and is not limited herein. The preset sparse model may analyze various diseases that require processing of gene sequencing data, such as bladder cancer, acute myocardial infarction, and acute myeloid leukemia. Disease categories analyzed by the preset sparse model are different, as are gene loci processed by the preset sparse model, and the number of the gene loci processed.

The preset sparse model is configured to process the target gene sequencing ciphertext and output the gene sequencing data analysis result ciphertext. The target gene sequencing ciphertext is a gene sequencing ciphertext including the gene locus data to be detected in the plurality of gene sequencing ciphertexts. For ease of understanding, assume the number of the gene sequencing ciphertexts is 84, and the number of gene loci to be detected is 9. Typically, when machine learning models other than sparse models perform computations, 84 gene sequencing ciphertexts need to participate in a calculation. In reality, there are at most 9 gene sequencing ciphertexts that contain the gene locus to be detected. Models other than sparse models need to process all the gene sequencing ciphertexts, resulting in low computational efficiency. Due to an application of model sparsity in the preset sparse model, model parameters corresponding to at least 84-9=75 gene sequencing ciphertexts are zero, meaning result ciphertexts corresponding to at least 75 gene sequencing ciphertexts are zero. At most, the result ciphertexts corresponding to 9 gene sequencing ciphertexts need to participate in a summation calculation to output the gene sequencing data analysis result ciphertext. Computational efficiency is significantly improved, thereby enhancing efficiency of gene sequencing data analysis.

Step S330, sending the gene sequencing data analysis result ciphertext to the user equipment, so that the user equipment decrypts the gene sequencing data analysis result ciphertext to determine a gene sequencing data analysis result of a user.

The server sends the gene sequencing data analysis result ciphertext to the user equipment. The user equipment receives the gene sequencing data analysis result ciphertext, and decrypts the gene sequencing data analysis result ciphertext to determine the gene sequencing data analysis result of the user. The preset sparse model, due to that most of the model parameters are zero, even when the user device sends the plurality of genomic sequencing ciphertexts, does not need to process all gene sequencing ciphertexts, thereby improving computational efficiency of the model, and efficiency of gene sequencing data analysis.

### Third Embodiment

Referring to FIG. 4, FIG. 4 is a first structural schematic diagram of a gene sequencing data analysis apparatus according to an embodiment of the present disclosure. The gene sequencing data analysis apparatus 400 in FIG. 4 is applied to a user equipment. The gene sequencing data analysis apparatus 400 includes:
a data encoding module 410, configured to encode gene sequencing data of a user to obtain a plurality of gene sequencing plaintexts corresponding to the gene sequencing data;
a plaintext encryption module 420, configured to encrypt each of the plurality of gene sequencing plaintexts using a public key to obtain a plurality of gene sequencing ciphertexts;
a ciphertext sending module 430, configured to send the plurality of gene sequencing ciphertexts to a server, where the server is configured to input the plurality of gene sequencing ciphertexts into a preset sparse model to obtain a gene sequencing data analysis result ciphertext;
a ciphertext receiving module 440, configured to receive the gene sequencing data analysis result ciphertext sent by the server; and
a result determination module 450, configured to decrypt the gene sequencing data analysis result ciphertext using a private key to determine a gene sequencing data analysis result of the user.

In the embodiments of the present disclosure, the gene sequencing data analysis apparatus 400 further includes:
a security parameter generation module, configured to generate, based on a ciphertext polynomial degree and a bit length of a ciphertext modulus of a homomorphic encryption, a security parameter; and
a public key and private key generation module, configured to generate the public key and the private key according to the security parameter.

In embodiments of the present disclosure, the data encoding module 410 includes:
an encoding dimension determination sub-module, configured to determine an encoding dimension corresponding to each of the plurality of gene sequencing ciphertexts according to the ciphertext polynomial degree; and
a plaintext acquisition sub-module, configured to encode, based on the encoding dimension and a dimension of the gene sequencing data of the user, the gene sequencing data, to obtain the plurality of gene sequencing plaintexts.

In the embodiments of the present disclosure, the gene sequencing data analysis apparatus 400 further includes:
a data padding module, configured to, when a dimension of the plurality of gene sequencing plaintexts is less than the encoding dimension, performing data padding on the plurality of gene sequencing plaintexts until the dimension of the plurality of gene sequencing plaintexts equals the encoding dimension.

In the embodiments of the present disclosure, a dimension of model parameters of the preset sparse model equals the dimension of the gene sequencing data, and the model parameters include model sub-parameters corresponding to each of the plurality of gene sequencing plaintexts; and
the preset sparse model is configured to obtain a plurality of result ciphertexts respectively according to the model sub-parameters and the plurality of gene sequencing ciphertexts, and sum the plurality of result ciphertexts to obtain the gene sequencing data analysis result ciphertext.

In embodiments of the present disclosure, the result determination module 450 includes:
a plaintext vector acquisition sub-module, configured to decrypt the gene sequencing data analysis result ciphertext using the private key to obtain a plaintext vector corresponding to the gene sequencing data analysis result, where the plaintext vector comprises a plurality of components;
a component acquisition sub-module, configured to obtain any one of the plurality of components from the plaintext vector;
an abnormality determination sub-module, configured to, when a numerical value of the component is positive, determine that the gene sequencing data analysis result of the user indicates the genetic sequencing data is abnormal; and
a no-abnormality determination sub-module, configured to, when the numerical value of the component is negative, determine that the gene sequencing data analysis result of the user indicates the genetic sequencing data is not abnormal.

In the embodiments of the present disclosure, the gene sequencing data analysis apparatus 400 further includes:
an analysis document generation module, configured to generate a data analysis and processing document of the user according to the gene sequencing data analysis result of the user; and
a display interface generation module, configured to convert the data analysis and processing document into a QR code image and generate a display interface for the QR code image.

The gene sequencing data analysis apparatus 400 is configured to execute the corresponding steps in the above-mentioned gene sequencing data analysis method. Specific implementation of each function may not be described in detail here. Furthermore, optional examples in the gene sequencing data analysis method are also applicable to the gene sequencing data analysis apparatus 400.

### Fourth Embodiment

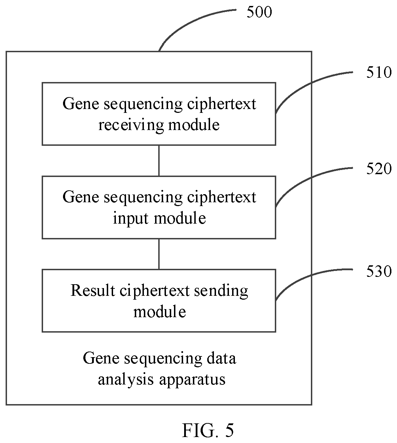

Referring to FIG. 5, FIG. 5 is a second structural schematic diagram of a gene sequencing data analysis apparatus according to an embodiment of the present disclosure. The gene sequencing data analysis apparatus 500 in FIG. 5 is applied to a server. The gene sequencing data analysis apparatus 500 includes:
a gene sequencing ciphertext receiving module 510, configured to receive a plurality of gene sequencing ciphertexts sent by a user equipment;
a gene sequencing ciphertext input module 520, configured to input the plurality of gene sequencing ciphertexts into a preset sparse model to obtain a gene sequencing data analysis result ciphertext, where the preset sparse model is configured to process a target gene sequencing ciphertext and output the gene sequencing data analysis result ciphertext, and the target gene sequencing ciphertext is a gene sequencing ciphertext including gene locus data to be detected in the plurality of gene sequencing ciphertexts; and
a result ciphertext sending module 530, configured to send the gene sequencing data analysis result ciphertext to the user equipment, so that the user equipment decrypts the gene sequencing data analysis result ciphertext to determine the gene sequencing data analysis result of the user.

This embodiment further provides a computing device, including:
a memory, configured to store instructions; and
a processor, configured to invoke the instructions from the memory and implement the above-mentioned gene sequencing data analysis method when executing the instructions.

In this embodiment, the data encoding module 410, the plaintext encryption module 420, the ciphertext sending module 430, the ciphertext receiving module 440, the result determination module 450, the gene sequencing ciphertext receiving module 510, the gene sequencing ciphertext input module 520, the result ciphertext sending module 530, etc., are stored as program units in the memory. Corresponding functions are implemented by the processor executing the above-mentioned program units stored in the memory.

The processor includes a kernel that retrieves the corresponding program units from the memory. One or more kernels may be configured, and the problem of easy leakage of user privacy during remote gene sequencing data analysis is addressed by adjusting kernel parameters.

The memory may include forms of non-permanent storage, Random Access Memory (RAM) and/or non-volatile memory in a computer-readable medium, such as Read-Only Memory (ROM) or flash RAM. The memory includes at least one memory chip.

The embodiments of the present disclosure further provide a machine-readable storage medium. The machine-readable storage medium stores instructions used to cause a machine to execute the above-mentioned gene sequencing data analysis method.

Those skilled in the art may understand that the embodiments of the present disclosure may be implemented as methods, devices, or computer program products. Therefore, the present disclosure may be implemented entirely in hardware, entirely in software, or as a combination of hardware and software aspects. Moreover, the present disclosure may take a form of the computer program product implemented on one or more computer-usable storage medium (including but not limited to disk storage, CD-ROM, optical storage, etc.) including computer-usable program code.

The present disclosure is described with reference to flowcharts and/or block diagrams of the method, device (system), and computer program product according to the embodiments of the present disclosure. It is understandable that each process and/or block in the flowcharts and/or the block diagrams, as well as combinations of the processes and/or the blocks in the flowcharts and/or the block diagrams, may be implemented by computer program instructions. These computer program instructions may be provided to the processor of a general-purpose computer, a special-purpose computer, an embedded processor, or other programmable data processing devices to produce a machine. The instructions, when executed by the processor of the computer or the other programmable data processing devices, create devices for implementing the functions specified in one or more processes of the flowcharts and/or one or more blocks of the block diagrams.

These computer program instructions may also be stored in a computer-readable memory that may direct the computer or the other programmable data processing device to function in a specific manner. The instructions stored in the computer-readable memory produce an article of manufacture that includes an instruction device implementing the functions specified in one or more processes of the flowchart and/or one or more blocks of the block diagram.

These computer program instructions may also be loaded onto the computer or the other programmable data processing device, causing a series of operational steps to be executed on the computer or the other programmable device to produce computer-implemented processing. Thus, the instructions executed on the computer or the other programmable device provide steps for implementing the functions specified in one or more processes of the flowchart and/or one or more blocks of the block diagram.

In a typical configuration, the computing device includes one or more processors (CPU), input/output interfaces, network interfaces, and memories.

The memory may include forms of non-permanent storage, Random Access Memory (RAM) and/or non-volatile memory in the computer-readable medium, such as Read-Only Memory (ROM) or flash RAM. The memory is an example of a computer-readable medium.

The machine-readable storage medium include permanent and non-permanent, removable and non-removable medium, and may be implemented by any method or technology for information storage. Information may be computer-readable instructions, data structures, program modules, or other data. Examples of computer storage medium include, but are not limited to, Phase-change Random Access Memory (PRAM), Static Random Access Memory (SRAM), Dynamic Random Access Memory (DRAM), other types of RAM, ROM, Electrically Erasable Programmable Read-Only Memory (EEPROM), flash memory or other memory technologies, Compact Disc Read-Only Memory (CD-ROM), Digital Versatile Disc (DVD) or other optical storage, magnetic cassettes, magnetic tape, magnetic disk storage or other magnetic storage devices, or any other non-transitory medium that may be configured to store information accessible by the computing device. According to the definition provided herein, the computer-readable medium does not include transitory medium, such as modulated data signals and carrier waves.

It should further be noted that the terms 'comprise', 'include' or any other variations thereof are intended to cover non-exclusive inclusion, meaning that a process, a method, a product, or a device including a series of elements includes not only those elements but also other elements not explicitly listed, or elements inherent to such the process, the method, the product, or the device. In the absence of more specific limitations, an element defined by the phrase "including a..." does not preclude the existence of additional identical elements in the process, the method, the product, or the device that includes the said element.

The above descriptions are merely the embodiments of the present disclosure and are not intended to limit the present disclosure. For those skilled in the art, the present disclosure may have various modifications and changes. Any modifications, equivalent replacements, improvements, etc., made within the spirit and principles of the present disclosure may be included within the scope of the claims of the present disclosure.

## Claims

1. A gene sequencing data analysis method, applied to a user equipment, wherein the gene sequencing data analysis method comprises:
encoding gene sequencing data of a user to obtain a plurality of gene sequencing plaintexts corresponding to the gene sequencing data;
encrypting each of the plurality of gene sequencing plaintexts using a public key to obtain a plurality of gene sequencing ciphertexts;
sending the plurality of gene sequencing ciphertexts to a server, wherein the server is configured to input the plurality of gene sequencing ciphertexts into a preset sparse model to obtain a gene sequencing data analysis result ciphertext;
receiving the gene sequencing data analysis result ciphertext sent by the server; and
decrypting the gene sequencing data analysis result ciphertext using a private key to determine a gene sequencing data analysis result of the user.

2. The gene sequencing data analysis method according to claim 1, wherein the gene sequencing data analysis method further comprises:
generating, based on a ciphertext polynomial degree and a bit length of a ciphertext modulus of a homomorphic encryption, a security parameter; and
generating the public key and the private key according to the security parameter.

3. The gene sequencing data analysis method according to claim 2, wherein the encoding the gene sequencing data of the user to obtain the plurality of gene sequencing plaintexts corresponding to the gene sequencing data, comprises:
determining an encoding dimension corresponding to each of the plurality of gene sequencing ciphertexts according to the ciphertext polynomial degree; and
encoding, based on the encoding dimension and a dimension of the gene sequencing data of the user, the gene sequencing data, to obtain the plurality of gene sequencing plaintexts.

4. The gene sequencing data analysis method according to claim 3, wherein the gene sequencing data analysis method further comprises:
when a dimension of the plurality of gene sequencing plaintexts is less than the encoding dimension, performing data padding on the plurality of gene sequencing plaintexts until the dimension of the plurality of gene sequencing plaintexts equals the encoding dimension.

5. The gene sequencing data analysis method according to claim 1, wherein a dimension of model parameters of the preset sparse model equals a dimension of the gene sequencing data, and the model parameters comprise model sub-parameters corresponding to each of the plurality of gene sequencing plaintexts; and
the preset sparse model is configured to obtain a plurality of result ciphertexts respectively according to the model sub-parameters and the plurality of gene sequencing ciphertexts, and sum the plurality of result ciphertexts to obtain the gene sequencing data analysis result ciphertext.

6. The gene sequencing data analysis method according to claim 1, wherein the decrypting the gene sequencing data analysis result ciphertext using the private key to determine the gene sequencing data analysis result of the user, comprises:
decrypting the gene sequencing data analysis result ciphertext using the private key to obtain a plaintext vector corresponding to the gene sequencing data analysis result, wherein the plaintext vector comprises a plurality of components;
obtaining any one of the plurality of components from the plaintext vector;
when a numerical value of the component is positive, determining that the gene sequencing data analysis result of the user indicates the genetic sequencing data is abnormal; and
when the numerical value of the component is negative, determining that the gene sequencing data analysis result of the user indicates the genetic sequencing data is not abnormal.

7. The gene sequencing data analysis method according to claim 1, wherein the gene sequencing data analysis method further comprises:
generating a data analysis and processing document of the user according to the gene sequencing data analysis result of the user; and
converting the data analysis and processing document into a QR code image and generating a display interface for the QR code image.

8. A gene sequencing data analysis method, applied to a server, wherein the gene sequencing data analysis method comprises:
receiving a plurality of gene sequencing ciphertexts sent by a user equipment;
inputting the plurality of gene sequencing ciphertexts into a preset sparse model to obtain a gene sequencing data analysis result ciphertext; wherein the preset sparse model is configured to process a target gene sequencing ciphertext and output the gene sequencing data analysis result ciphertext, and the target gene sequencing ciphertext is a gene sequencing ciphertext comprising gene locus data to be detected in the plurality of gene sequencing ciphertexts; and
sending the gene sequencing data analysis result ciphertext to the user equipment, so that the user equipment decrypts the gene sequencing data analysis result ciphertext to determine a gene sequencing data analysis result of a user.

9. A gene sequencing data analysis apparatus, applied to a user equipment, wherein the gene sequencing data analysis apparatus comprises:
a data encoding module, configured to encode gene sequencing data of a user to obtain a plurality of gene sequencing plaintexts corresponding to the gene sequencing data;
a plaintext encryption module, configured to encrypt each of the plurality of gene sequencing plaintexts using a public key to obtain a plurality of gene sequencing ciphertexts;
a ciphertext sending module, configured to send the plurality of gene sequencing ciphertexts to a server, wherein the server is configured to input the plurality of gene sequencing ciphertexts into a preset sparse model to obtain a gene sequencing data analysis result ciphertext;
a ciphertext receiving module, configured to receive the gene sequencing data analysis result ciphertext sent by the server; and
a result determination module, configured to decrypt the gene sequencing data analysis result ciphertext using a private key to determine a gene sequencing data analysis result of the user.

10. A gene sequencing data analysis apparatus, applied to a server, wherein the gene sequencing data analysis apparatus comprises:
a gene sequencing ciphertext receiving module, configured to receive a plurality of gene sequencing ciphertexts sent by a user equipment;
a gene sequencing ciphertext input module, configured to input the plurality of gene sequencing ciphertexts into a preset sparse model to obtain a gene sequencing data analysis result ciphertext; wherein the preset sparse model is configured to process a target gene sequencing ciphertext and output the gene sequencing data analysis result ciphertext, and the target gene sequencing ciphertext is a gene sequencing ciphertext comprising gene locus data to be detected in the plurality of gene sequencing ciphertexts; and
a result ciphertext sending module, configured to send the gene sequencing data analysis result ciphertext to the user equipment, so that the user equipment decrypts the gene sequencing data analysis result ciphertext to determine the gene sequencing data analysis result of the user.

11. A computing device, comprising:
a memory, configured to store instructions; and
a processor, configured to invoke the instructions from the memory and implement the gene sequencing data analysis method according to any one of claims 1 to 7 or claim 8 when executing the instructions.

12. A machine-readable storage medium, wherein the machine-readable storage medium stores instructions and the instructions are used to cause a machine to execute the gene sequencing data analysis method according to any one of claims 1 to 7 or claim 8.
